# EUROPEAN PATENT SPECIFICATION

(11) **EP 4 142 862 B1**
(45) Date of publication and mention of the grant of the patent: **18.03.2026**
(21) Application number: 21795430.4
(22) Date of filing: 28.04.2021
(51) Int. Cl.: A61N 1/05, A61N 1/36, A61B 18/14

(54) **MINIMALLY-INVASIVE ELECTRODE ARRAY FOR SPINAL CORD AND BRAIN STIMULATION WITH SHAPE-MEMORY ALLOY AND/OR POLYMER**
MINIMALINVASIVES ELEKTRODEN-ARRAY ZUR STIMULATION DES RÜCKENMARKS UND DES GEHIRNS MIT FORMDRANGLEGIERUNG UND/ODER POLYMER
RÉSEAU D'ÉLECTRODES MINIMALEMENT INVASIF POUR STIMULER LA MOELLE ÉPINIÈRE ET LE CERVEAU AVEC UN ALLIAGE À MÉMOIRE DE FORME ET/OU UN POLYMÈRE

(30) Priority: 28.04.2020 US 202063016716 P; 27.10.2020 US 202063106062 P
(43) Date of publication of application: 08.03.2023
(73) Proprietor: Brown University, Providence, RI 02912 (US); Rhode Island Hospital, Providence, RI 02903 (US)
(72) Inventor: TELFEIAN, Albert, Providence, RI 02906 (US); SRIVASTAVA, Vikas, East Greenwich, RI 02818 (US); SAMPATH, Shailen, Lincoln, RI 02865 (US)
(74) Representative: Wilson Gunn
(86) International application number: PCT/US2021/029704
(87) International publication number: WO 2021/222446

(56) References cited:
- WO-A1-2020/036886
- AU-A1- 2016 210 644
- US-A- 5 662 621
- US-A- 5 662 621
- US-A- 5 782 239
- US-A- 6 050 992
- US-A1- 2003 032 997
- US-A1- 2008 081 881
- US-A1- 2008 140 153
- US-A1- 2008 140 153
- US-A1- 2010 114 272
- US-A1- 2019 008 456
- US-B1- 7 181 288
- US-B2- 6 745 079

## Description

### BACKGROUND OF THE INVENTION

The present invention relates generally to electrode arrays, and more particularly to a shape-memory alloy and polymer electrode array for minimally-invasive spinal cord and brain stimulation and recording.

In general, it is advantageous to have an electrode system for spine and brain stimulation and recording that can be inserted using a minimally invasive procedure and that can achieve high coverage of intended neuronal targets internally after deployment. However, such electrode systems have been slow to develop, partially due to problems with the materials used in existing metallic and ceramic probes. As a result, most methods to stimulate and record neuronal activity cause unnecessary tissue damage.
U.S. Patent No. 5,782,239 describes an electrophysiological mapping device which includes an outer catheter, an inner catheter that is slidable within the outer catheter, and an electronic activation and recording device for electrically activating electrodes on the inner catheter and/or recording electric signals received by the electrodes.
AU2016210644 describes a catheter having a basket-shaped electrode assembly with a high electrode density.
U.S. Pat. No. 6,745,079 describes an implantable lead for electrical stimulation of tissue which has wire-like extendable members whose tips curl back upon themselves in open tissue spaces to form 2- or 3-dimensional electrodes.

### SUMMARY OF THE INVENTION

The invention is defined in claim 1.

The following presents a simplified summary of the innovation in order to provide a basic understanding of some aspects of the invention. This summary is not an extensive overview of the invention. It is intended to neither identify key or critical elements of the invention nor delineate the scope of the invention. Its sole purpose is to present some concepts of the invention in a simplified form as a prelude to the more detailed description that is presented later.

In an aspect, the invention features a minimally-invasive electrode array for bodily insertion that is capable to adapt to a minimally-invasive shape and to move to a shape to maximize a coverage area, comprising: a central lumen surrounded by an insulation layer, the central lumen including one or more elastic and/or super-elastic alloys and/or shape-memory polymers and/or alloys with an initially programmed shape, the insulation layer including a plurality of orbital lumens positioned in a ring shape, the orbital lumens being separated from each other by insulation of the insulation layer, each of the orbital lumens configured to carry at least one conductive component; and a removable outer sheath surrounding the insulation layer and operative to provide a shape change, the removable outer sheath capable of holding the electrode array into an insertion shape or introducer shape or geometry when surrounding the insulation layer, and a central lumen surrounded by the insulation layer and capable of reverting the electrode array to the initially programmed shape after removal of the outer sheath.

The central lumen may further include a thermoset shape-memory polymer.

There is also described a method, not encompassed by the wording of the claims, including adapting biocompatible shape-memory alloy-based wires to an electrode array, inserting the adapted electrode array in a minimally invasive fashion through a needle into a part of a human body, and transforming the adapted electrode array electrode shape using heat to maximize a coverage area of the part of the human body.

These and other features and advantages will be apparent from a reading of the following detailed description and a review of the associated drawings. It is to be understood that both the foregoing general description and the following detailed description are explanatory only and are not restrictive of aspects as claimed.

### BRIEF DESCRIPTION OF THE DRAWINGS

These and other features, aspects, and advantages of the present invention will become better understood with reference to the following description, appended claims, and accompanying drawings where:
FIG. 1 illustrates a cross-section of an exemplary modified cylindrical lead.
FIG. 2 illustrates an exemplary view of the three steps in inserting a modified cylindrical lead for dorsal column stimulation into the epidural space that can allow for minimally invasive insertion of an electrode with more coverage.
FIG. 3 illustrates three exemplary examples of how the heat induced shape memory ability of our modified electrode could be activated.
FIG. 4 illustrates the exemplary thermally induced shape memory ability of shape memory polymers.
FIG. 5 illustrates the exemplary shape memory ability of Nitinol as it is deformed into a temporary shape and heated to allow for recovery to its original geometry in four steps.
FIG. 6 illustrates two exemplary example geometries that a nitinol component of a cylindrical lead can take.

### DETAILED DESCRIPTION

The subject innovation is now described with reference to the drawings, wherein reference numerals are used to refer to like elements throughout. In the following description, for purposes of explanation, numerous specific details are set forth in order to provide a thorough understanding of the present invention. It may be evident, however, that the present invention may be practiced without these specific details. In other instances, well-known structures and devices are shown in block diagram form in order to facilitate describing the present invention.

Neuromodulation is a rapidly expanding field in clinical neuroscience. Today, electrode arrays in the form of leads, grids, and paddles are placed to stimulate and record in the brain and spinal cord for the treatment of neurologic and psychiatric diseases that include, for example, epilepsy, Parkinson's, obsessive-compulsive disorder and pain. In order to maximize electrode coverage in the brain and the spine, typically, a craniotomy or laminectomy must be performed in order to place a large grid or paddle electrode to cover the brain or spine.

The present invention is an adaption to traditional electrode arrays: biocompatible shape-memory alloy and/or thermoset and thermoplastic polymer based wires, sheathings, hinges or other compact geometries are used to insert an electrode array in a minimally invasive fashion through a needle or a small window in the spine or skull and then using the shape memory response of the shape memory alloy and/or polymer to transform that electrode shape into a shape that covers a more desirable area of the brain or spinal cord. In our design, the use of elasticity of metal wires can provide higher level of shape changing force where needed. The shape changes of biocompatible shape memory alloys and polymers are mainly activated through thermal response. When the components reach above a certain transition temperature range a shape change is induced.

By way of one example, shape memory alloys can be used to optimize dorsal column stimulation. Dorsal column stimulation for chronic pain is a neuromodulation procedure whereby an electrode array can be placed on the dorsal columns of the spinal cord either percutaneously by sliding small straight electrodes through a needle and advancing them epidurally using X-ray over the appropriate spinal level, or through an open procedure where an electrode paddle is placed through an open laminectomy over the appropriate level. The present invention bridges a gap between the open and percutaneous procedure. The shape-memory alloy or polymer-based electrode system of the present invention can be placed percutaneously but have the same superior coverage of the spine level for stimulator that an open paddle electrode does. In the case of the shape memory alloy Nitinol, Nitinol is set to a desired shape ex vivo and deformed into a linear shape before insertion. Nitinol is able to retain its linear deformed shape outside the environment of the body. It's special characteristics as a thermally induced shape memory alloy allow it to maintain its shape of deformation when it is underneath its transition temperature. After the shape memory alloy Nitinol is placed in an environment above its transition temperature the material will exhibit superelastic characteristics and revert to its desired initially set shape. Therefore, once the Nitinol lead is inserted into the body it will revert to its initial programmed shape meant to optimize the leads coverage for dorsal column stimulation. The phase change that occurs below Nitinol's shape transition temperature and also once Nitinol is heated above it's transition temperature. The present invention can use body temperature as a natural way to heat the shape memory alloy and polymer above it's shape transitioning temperature without a need for external heat source.

In one embodiment, the present invention uses the shape memory effects of Nitinol, a metal alloy of nickel and titanium, or other shape memory alloy or shape memory polymer, to create a modified cylindrical lead that will expand to more closely resemble area coverage of a paddle lead or geometry that best targets specific dermatomes dictated by industrial preference, once it is exposed to the body's environment.

The modified cylindrical lead can be inserted in a minimally invasive fashion utilizing a percutaneous technique similar to cylindrical lead placement, before expanding in situ. The modified cylindrical lead provides more coverage than current cylindrical leads, reduces the need for costly and invasive laminectomies, and increases accessibility to the procedure to not only spine surgeons but all pain management physicians.

The modified cylindrical lead also can be used for applications beyond dorsal column targeted spinal cord stimulation, including stimulation of a peripheral nerve, stimulation of the dorsal root, and recording of nerves. In one implementation, this is achieved by utilizing the shape memory alloy Nitinol (NiTi).

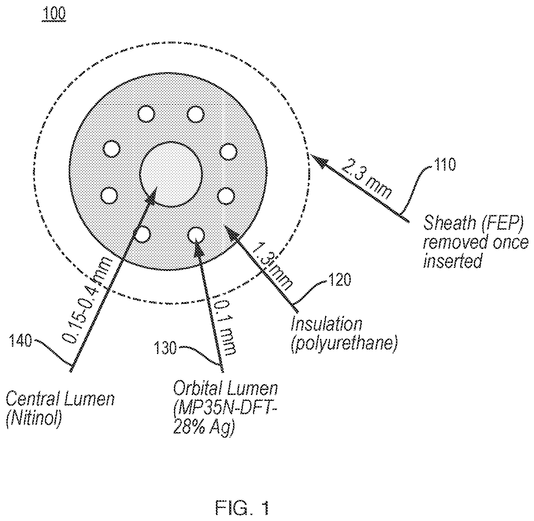

In FIG. 1, a cross-section of an exemplary modified cylindrical lead 100 for dorsal column stimulation is illustrated and includes an outer sheath 110 that is removed when the modified cylindrical lead 100 is inserted. Within the outer sheath 110 is an insulation layer 120 that includes a number of orbital lumen 130 that carry conductive components that deliver the stimulation. The insulation layer 120 surrounds a central lumen 140 that in one implementation uses the shape memory alloy Nitinol. In another implementation the central lumen 140 can have other shape memory alloys or polymers.

As described above, Nitinol (NiTi), is a metal alloy used in many medical applications due to its biocompatibility and super-elasticity. The present invention uses shape memory effect where the material undergoes a 'phase change' above a certain temperature that transitions the material from a material phase with a lower stiffness into a stiffer super-elastic material that takes on the original shape that it was processed in. Below body temperature, the modified cylindrical lead 100 can be manufactured in a shape that resembles a paddle lead spinal coverage, or any shape that would most optimally target a desired region. In the geometries produced, the modified cylindrical lead 100 is straightened, placed into an introducer sheath, and inserted into the epidural space percutaneously similar to a cylindrical electrode. Once inserted to the desired location, the sheath is removed and the Nitinol upon reaching body temperature reverts the lead back into a shape that provides optimal coverage.

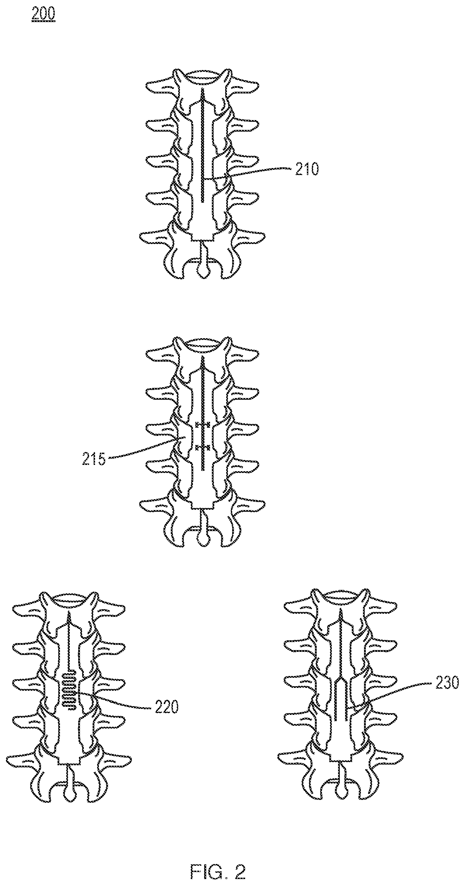

In Fig. 2, an exemplary representation of insertion of the modified cylindrical lead for dorsal column stimulation is illustrated. At 210, a strait modified cylindrical lead is inserted percutaneously. At 215, the lead is navigated up the epidural space to its desired region. At 220, the introducer sheath is removed and the modified electrode is allowed to revert the shape 230 it was processed in. At 220, the shape is in a s-shaped zig zag geometry. For demonstration purposes, here we illustrate two cylindrical leads 230, but more than two leads can be used in our application.

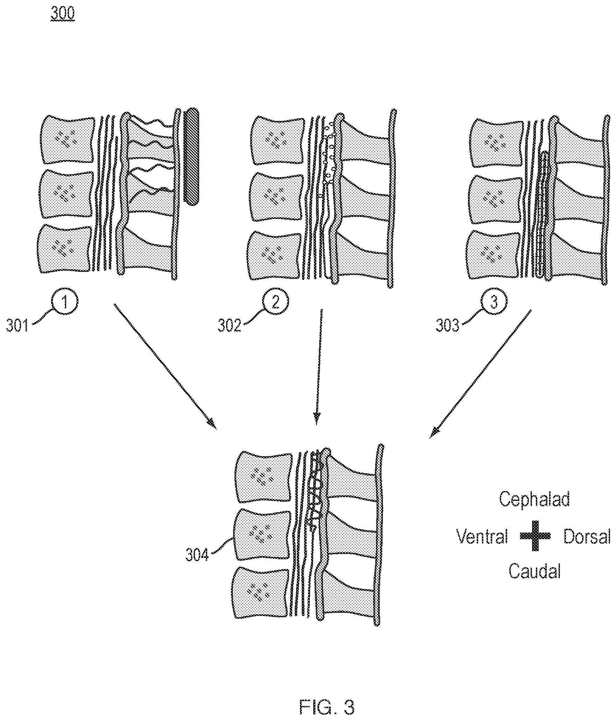

In Fig. 3, an exemplary representation 300 of how shape change of a modified cylindrical electrode for dorsal column stimulation in the body can be induced is illustrated. The recovery of the shape memory alloy or polymer is not restricted to heating the components to body temperature. The shape memory alloy and polymer can be synthesized and processed in a way which allows for customizability in the temperature range which induces a phase change.

In Fig. 3, an external heat source 301 can be used to induce shape change of the modified electrode in certain designs. In 302, cold saline water is administered along with the electrode which prevents recovery. In yet another example 303, a sheath can be used to prevent premature recovery of the modified electrode. These are exemplary methods that can be used to allow control over when shape recovery occurs 304.

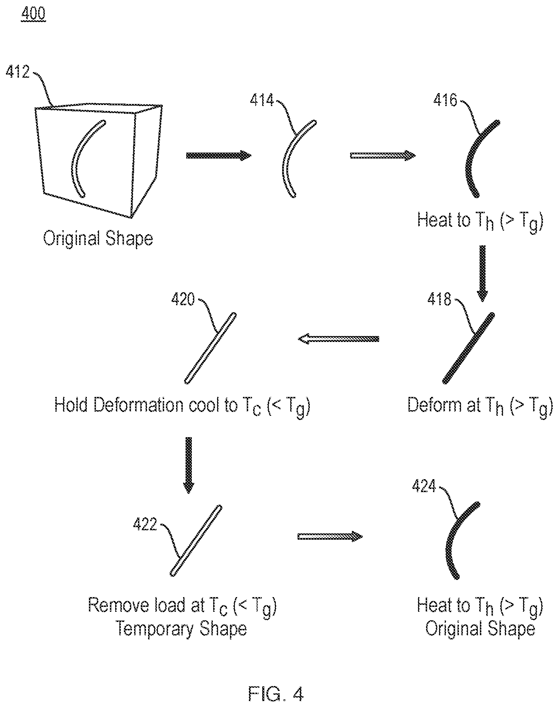

In FIG. 4, an exemplary representation of shape memory hinge fabrication 400 is shown. A thermoset shape-memory polymer 412 in its original shape 414 is heated to a high temperature Tₕ (> T_{g}) to provide a heated shape 416, where T_{g} is its glass transition temperature. The heated shape 416 is deformed to a deformed (straight) shape 418 at Tₕ (> T_{g}). The deformed shape 418 is cooled to a lower temperature T_{c} (> T_{g}) to hold a shape 420. Removing the load at T_{c} (> T_{g}) preserves the temporary shape 422. The shape 422 can be inserted through minimally invasive procedures and a body temperature or external stimulus can change the shape of hinge. Heating the temporary shape 422 at Tₕ (> T_{g}) returns the temporary shape 422 to its original thermoset shape-memory polymer shape 424.

In summary, when the original shape is brought to a temperature above the glass transition temperature, it is able to deform in its rubbery soft state. The deformation can then be held when the material is cooled. The polymer returns to its original shape when heated above glass transition temperature. The same process applies for a shape-memory alloy changing shape to a desired shape.

In one specific example, a shape memory polymer is made using Tert-butyl acrylate (tBA), poly(ethylene glycol)n dimethacrylate (PEGDMA), and photoinitiater 2,2- dimethoxy-2-phenylacetophenone. The constituents are 89.2%, 10%, and 0.2% by weight, respectively. Shape memory polymer sheets are made via mold of a petri dish and top made of glass slides, in which the mixed and autoclaved (for 10 minutes) solution is placed in between the two glass surfaces. This mold is separated by 1-2 spacers also made of glass slides, each 0.15 inches (3.175 mm) in thickness. Afterwards the solution is UV cured for 15 minutes and oven cured for 60 minutes at 90° C to get the final shape memory polymer in sheet form. The semicircular shaped hinges with rectangular cross section are then cut from this sheet.

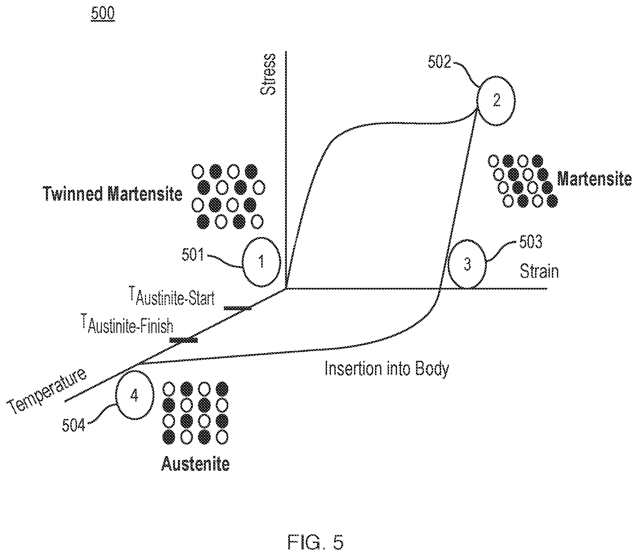

In FIG. 5, an exemplary illustration 500 of the phase change of Nitinol is illustrated. At 501, the Nitinol is in a twinned martensite shape at temperatures below a specific transition temperature range. Below the transition range in a twinned martensite phase, Nitinol can be easily deformed at 502 up to 11% strain into another geometry, such as a linear shape for easier percutaneous insertion of an electrode. This will cause Nitinol to go from a twinned martensite to a martensite phase, which will maintain its 'deformed' shape at 503 until heated above its transition range. Once Nitinol is heated about its transition temperature it will convert to an austinite phase at 504 and will take the shape it was in when it was in its twinned martensite phase.

To obtain the original austinite shape of the Nitinol, the Nitinol can either be obtained in the shape that is desired, machined in the desired shape from a blanket form, or commercially available Nitinol can be set to our desired shape. To set the austinite shape of the Nitinol, the material must be heated to 500 °C for one to three hours fixed in the shape for intended use.

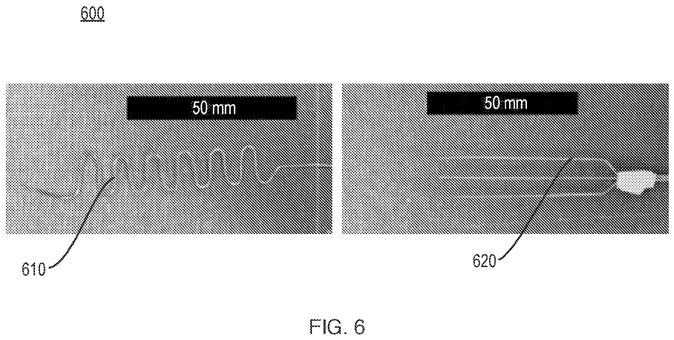

In FIG. 6, exemplary images of example geometries of a nitinol component of the cylindrical lead are illustrated. Image 610 displays a potential s-shaped geometry. Image 620 displays a trident shaped geometry. This geometry could have as few as two and as many parallel cylindrical leads as wanted that would break apart from each other when exposed to the bodies environment and allowed to recover. These geometries are examples and are not exhaustive of all the potential geometries the shape memory alloy and polymer based cylindrical leads could take.

## Claims

1. A minimally-invasive electrode array for bodily insertion that is capable to adapt to a minimally-invasive shape and to move to a shape to maximize a coverage area, comprising:
a central lumen (140) surrounded by an insulation layer (120), the central lumen (140) including one or more elastic and/or super-elastic alloys and/or shape-memory polymers and/or alloys with an initially programmed shape, the insulation layer (120) including a plurality of orbital lumens (130) positioned in a ring shape, the orbital lumens being separated from each other by insulation of the insulation layer, each of the orbital lumens configured to carry at least one conductive component; and
a removable outer sheath (110) surrounding the insulation layer (120) and operative to provide a shape change, the removable outer sheath (110) capable of holding the electrode array into an insertion shape or introducer shape or geometry when surrounding the insulation layer (120) and capable of reverting the electrode array to the initially programmed shape after a removal of the outer sheath (110).

2. The minimally-invasive electrode array of claim 1, wherein the removable outer sheath (110) is capable of a sufficient flexibility that is capable to move or to accommodate when the one or more elastic and/or super-elastic alloys and/or shape-memory polymers with an initially programmed shape move from an insertion shape or introducer shape or geometry to the initially programmed shape with said movement caused by a shape change to the initially programmed shape.

3. The minimally-invasive electrode array of claim 1, wherein the removal or a withdrawal of the outer sheath (110) is capable of moving the minimally-invasive electrode array from the insertion shape or introducer shape or geometry to the initially programmed shape after the removal of the outer sheath (110); and wherein the initially programmed shape is a shape operative to be useful for a surgery that could not be obtained in a minimally-invasive electrode array that does not include the removable outer sheath operative to provide a shape change.

4. The minimally-invasive electrode array of claim 1, wherein the central lumen (140) further comprises a metal alloy of nickel and titanium.

5. The minimally-invasive electrode array of claim 1, wherein the central lumen (140) further includes a thermoset shape-memory polymer, and wherein the one or more elastic and/or super-elastic alloys comprise one or more elastic and/or super-elastic metal wires with an initially programmed shape.

6. The minimally-invasive electrode array of claim 1, wherein the removable outer sheath (110) comprises fluorinated ethylene propylene (FEP); wherein the removable outer sheath (110) further comprises a wire.

7. The minimally-invasive electrode array of claim 1, wherein the insulation layer (120) comprises polyurethane.

8. The minimally-invasive electrode array of claim 1, wherein the shape-memory polymers and/or the alloys with the initially programmed shape comprise, in weight percent based on a total weight of the shape-memory polymers and/or the alloys with the initially programmed shape:
89.2% Tert-butyl acrylate (tBA);
10% poly(ethylene glycol)n dimethacrylate (PEGDMA); and
0.2% photoinitiater 2,2-dimethoxy-2-phenylacetophenone.

## Patentansprüche

1. Eine Minimalinvasives Elektrodenanordnung zum Einführen in den Körper, das sich an eine minimalinvasive Form anpassen und zu einer Form bewegen kann, um einen Abdeckungsbereich zu maximieren, umfassend:
ein zentrales Lumen (140), das von einer Isolierschicht (120) umgeben ist, wobei das zentrale Lumen (140) eine oder mehrere elastische und/oder superelastische Legierungen und/oder Formgedächtnispolymere und/oder Legierungen mit einer anfänglich programmierten Form enthält, wobei die Isolierschicht (120) eine Vielzahl von ringförmig angeordneten orbitalen Lumina (130) enthält, wobei die orbitalen Lumina voneinander durch die Isolierung der Isolierschicht getrennt sind, jedes der orbitalen Lumina so konfiguriert ist, dass es mindestens eine leitfähige Komponente trägt; und
eine die Isolierschicht (120) umgebende und zum Bereitstellen einer Formänderung wirksame entfernbare Außenhülle (110), wobei die entfernbare Außenhülle (110) in der Lage ist, die Elektrodenanordnung in eine Einbringungsform oder Einführform oder -Geometrie zu halten, wenn sie die Isolierschicht (120) umgibt, und in der Lage ist, die Elektrodenanordnung nach einem Entfernen der Außenhülle (110) in die anfänglich programmierte Form zurückzubringen.

2. Die Minimalinvasive Elektrodenanordnung nach Anspruch 1, wobei die entfernbare Außenhülle (110) zu einer ausreichenden Flexibilität fähig ist, die fähig ist, sich zu bewegen oder anzupassen, wenn sich die eine oder mehreren elastischen und/oder superelastischen Legierungen und/oder Formgedächtnispolymere mit einer anfänglich programmierten Form von einer Einbringungsform oder Einführform oder - Geometrie in die anfänglich programmierte Form bewegen, wobei die besagte Bewegung durch eine Formänderung in die anfänglich programmierte Form verursacht wird.

3. Die Minimalinvasive Elektrodenanordnung nach Anspruch 1, wobei das Entfernen oder ein Zurückziehen der Außenhülle (110) fähig ist, die minimalinvasive Elektrodenanordnung nach dem Entfernen der Außenhülle (110) von der Einbringungsform oder Einführform oder Geometrie in die ursprünglich programmierte Form zu bewegen; und wobei die ursprünglich programmierte Form eine Form ist, die wirksam ist, für eine Operation nützlich zu sein, die mit einer minimalinvasiven Elektrodenanordnung, die keine zur Bereitstellung einer Formänderung wirksame entfernbare Außenhülle umfasst, nicht erreicht werden könnte.

4. Die Minimalinvasive Elektrodenanordnung nach Anspruch 1, wobei das zentrale Lumen (140) ferner eine Metalllegierung aus Nickel und Titan umfasst.

5. Die Minimalinvasive Elektrodenanordnung nach Anspruch 1, wobei das zentrale Lumen (140) ferner ein thermisch ausgehärtetes Formgedächtnispolymer umfasst und wobei die eine oder mehreren elastischen und/oder superelastischen Legierungen einen oder mehrere elastische und/oder superelastische Metalldrähte mit einer anfänglich programmierten Form umfassen.

6. Die Minimalinvasive Elektrodenanordnung nach Anspruch 1, wobei die entfernbare Außenhülle (110) fluoriertes Ethylenpropylen (FEP) umfasst; wobei die entfernbare Außenhülle (110) ferner einen Draht umfasst.

7. Die Minimalinvasive Elektrodenanordnung nach Anspruch 1, wobei die Isolierschicht (120) Polyurethan umfasst.

8. Die Minimalinvasive Elektrodenanordnung nach Anspruch 1, wobei die Formgedächtnispolymere und/oder die Legierungen mit der anfänglich programmierten Form, bezogen auf das Gesamtgewicht der Formgedächtnispolymere und/oder der Legierungen mit der anfänglich programmierten Form, in Gewichtsprozent umfassen:
89,2 % tert-Butylacrylat (tBA);
10 % Poly(ethylenglycol)n-dimethacrylat (PEGDMA); und
0,2 % Photoinitiator 2,2-Dimethoxy-2-phenylacetophenon.

## Revendications

1. Matrice d'électrodes mini-invasives pour insertion corporelle qui est capable de s'adapter à une forme mini-invasive et de se déplacer vers une forme pour maximiser une zone de couverture, comprenant :
une lumière centrale (140) entourée d'une couche d'isolation (120), la lumière centrale (140) comportant un ou plusieurs alliages élastiques et/ou super-élastiques et/ou polymères et/ou alliages à mémoire de forme avec une forme initialement programmée, la couche d'isolation (120) comportant une pluralité de lumières orbitales (130) positionnées en forme d'anneau, les lumières orbitales étant séparées les unes des autres par isolation de la couche d'isolation, chacune des lumières orbitales étant configurée pour porter au moins un composant conducteur ; et
une gaine externe (110) amovible entourant la couche d'isolation (120) et opérationnelle pour fournir un changement de forme, la gaine externe (110) amovible étant capable de maintenir la matrice d'électrodes dans une forme d'insertion ou forme ou géométrie d'introduction lorsqu'elle entoure la couche d'isolation (120) et étant capable de rétablir la matrice d'électrodes dans la forme initialement programmée après un enlèvement de la gaine externe (110).

2. Matrice d'électrodes mini-invasives selon la revendication 1, dans laquelle la gaine externe (110) amovible est capable de présenter une flexibilité suffisante qui est capable de se déplacer ou de s'adapter lorsque les un ou plusieurs alliages élastiques et/ou super-élastiques et/ou polymères à mémoire de forme avec une forme initialement programmée se déplacent d'une forme d'insertion ou forme ou géométrie d'introduction à la forme initialement programmée avec ledit mouvement causé par un changement de forme vers la forme initialement programmée.

3. Matrice d'électrodes mini-invasives selon la revendication 1, dans laquelle l'enlèvement ou un retrait de la gaine externe (110) est capable de déplacer la matrice d'électrodes mini-invasives de la forme d'insertion ou forme ou géométrie d'introduction à la forme initialement programmée après l'enlèvement de la gaine externe (110) ; et dans laquelle la forme initialement programmée est une forme opérationnelle pour servir à une chirurgie qui ne pourrait pas être obtenue dans une matrice d'électrodes mini-invasives qui ne comporte pas la gaine externe amovible opérationnelle pour fournir un changement de forme.

4. Matrice d'électrodes mini-invasives selon la revendication 1, dans laquelle la lumière centrale (140) comprend en outre un alliage métallique de nickel et de titane.

5. Matrice d'électrodes mini-invasives selon la revendication 1, dans laquelle la lumière centrale (140) comporte en outre un polymère à mémoire de forme thermodurci, et dans laquelle les un ou plusieurs alliages élastiques et/ou super-élastiques comprennent un ou plusieurs fils métalliques élastiques et/ou super-élastiques avec une forme initialement programmée.

6. Matrice d'électrodes mini-invasives selon la revendication 1, dans laquelle la gaine externe (110) amovible comprend de l'éthylène propylène fluoré (FEP) ; dans laquelle la gaine externe (110) amovible comprend en outre un fil.

7. Matrice d'électrodes mini-invasives selon la revendication 1, dans laquelle la couche d'isolation (120) comprend du polyuréthane.

8. Matrice d'électrodes mini-invasives selon la revendication 1, dans laquelle les polymères à mémoire de forme et/ou les alliages avec la forme initialement programmée comprennent, en pourcentage en poids sur la base d'un poids total des polymères à mémoire de forme et/ou des alliages avec la forme initialement programmée :
89,2 % d'acrylate de tert-butyle (tBA) ;
10 % de poly(éthylène glycol)n diméthacrylate (PEGDMA) ; et
0,2 % de photoinitiateur 2,2-diméthoxy-2-phénylacétophénone.
